# EUROPEAN PATENT SPECIFICATION

(11) **EP 2 581 053 B1**
(45) Date of publication and mention of the grant of the patent: **20.07.2016**
(21) Application number: 12188017.3
(22) Date of filing: 10.10.2012
(51) Int. Cl.: A61B 17/32

(54) **Surgical instrument with ultrasonic waveguide defining a fluid lumen**
Chirurgisches Instrument mit Ultraschallwellenleiter, der ein Flüssigkeitslumen definiert
Instrument chirurgical avec guide d'onde ultrasonore définissant un passage de fluide

(30) Priority: 10.10.2011 US 201113269894
(43) Date of publication of application: 17.04.2013
(73) Proprietor: Ethicon Endo-Surgery, Inc., Cincinnati, OH 45242 (US)
(72) Inventor: Stulen, Foster B., Mason, OH Ohio OH 45040 (US); Willis, John W., Cincinnati, OH Ohio OH 45244 (US); Dietz, Timothy G., Terrace Park, OH Ohio OH 45174 (US)
(74) Representative: Carpmaels & Ransford LLP

(56) References cited:
- WO-A1-00/00096
- US-A- 4 169 984
- US-A- 5 371 429

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient.

Examples of endoscopic surgical instruments include those disclosed in U.S. Pat. Pub. No. 2006/0079874, entitled "Tissue Pad Use with an Ultrasonic Surgical Instrument," published April 13, 2006, U.S. Pat. Pub. No. 2007/0191713, entitled "Ultrasonic Device for Cutting and Coagulating," published August 16, 2007, U.S. Pat. Pub. No. 2007/0282333, entitled "Ultrasonic Waveguide and Blade," published December 6, 2007, U.S. Pat. Pub. No. 2008/0200940, entitled "Ultrasonic Device for Cutting and Coagulating," published August 21, 2008, U.S. Pat. Pub. No. 2011/0015660, entitled "Rotating Transducer Mount for Ultrasonic Surgical Instruments," published January 20, 2011, U.S. Pat. No. 6,500,176, entitled "Electrosurgical Systems and Techniques for Sealing Tissue," issued December 31, 2002, and U.S. Pat. Pub. No. 2011/0087218, entitled "Surgical Instrument Comprising First and Second Drive Systems Actuatable by a Common Trigger Mechanism," published April 14, 2011 Additionally, such surgical tools may include a cordless transducer such as that disclosed in U.S. Pat. Pub. No. 2009/0143797, entitled "Cordless Hand-held Ultrasonic Cautery Cutting Device," published June 4, 2009.

Various kinds of surgical instruments may also be used, or adapted for use, in robotic-assisted surgery settings such as that disclosed in U.S. Pat. No. 6,783,524, entitled "Robotic Surgical Tool with Ultrasound Cauterizing and Cutting Instrument," issued August 31, 2004. Some versions of ultrasonic surgical instruments may further include structures to provide irrigation at a surgical site. Examples of such capabilities are described in U.S. Pat. No. 5,188,102, entitled "Surgical Ultrasonic Horn," issued February 23, 1 993. Additional examples of ultrasonic surgical instruments with fluid dispensation capabilities are disclosed in U.S. Pub. No. 2011/0152759, entitled "Use of Biomarkers and Therapeutic Agents with Surgical Devices," published June 23, 2011.

US 4,169,984 represents the closest prior art and discloses an ultrasonic surgical probe comprising a piezoelectric crystal transducer assembly positioned in compression between a pair of body members in a housing, wherein each body member along with the transducer assembly is mounted on a hollow connecting rod. The invention is defined in the appended claims.

### BRIEF DESCRIPTION OF THE FIGURES

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an exemplary surgical system comprising a surgical instrument and a generator;
FIG. 2 depicts a partial side elevation view of an exemplary surgical instrument with a portion of a cover removed to show the interior of a mating housing portion of an exemplary multi-piece handle assembly;
FIG. 3 depicts a partial perspective view of a distal end of an exemplary transducer;
FIG. 4 depicts a perspective view of an exemplary transmission assembly;
FIG. 5 depicts a perspective view of an exemplary alternative surgical system comprising a surgical instrument, a generator, a vacuum source, and a fluid source;
FIG. 6 depicts a perspective view of the end effector of the surgical instrument of FIG. 5;
FIG. 7 depicts a cross-sectional view of a waveguide, transducer, and fluid coupling of the surgical instrument of FIG. 5;
FIG. 8 depicts a partial cross-sectional view of an exemplary alternative interface between an ultrasonic horn and a hollow waveguide; and
FIG. 9 depicts a partial cross-sectional view of an exemplary alternative interface between a fluid conduit and a hollow waveguide.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. Overview of Exemplary Ultrasonic Surgical System

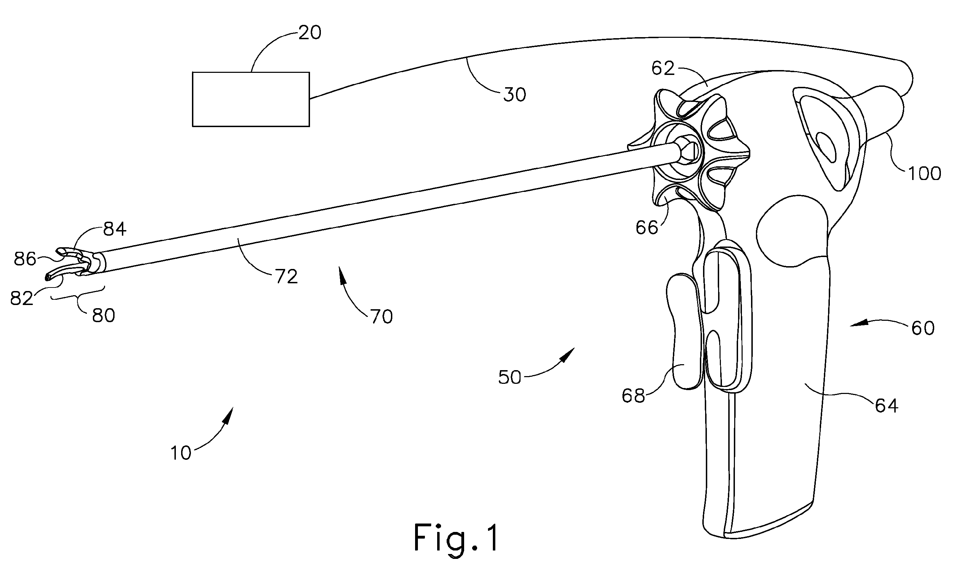

FIG. 1 shows an exemplary ultrasonic surgical system (10) comprising an ultrasonic surgical instrument (50), a generator (20), and a cable (30) coupling generator (20) to surgical instrument (50). In some versions, generator (20) comprises a GEN 300 sold by Ethicon Endo-Surgery, Inc. of Cincinnati, Ohio. By way of example only, generator (20) may be constructed in accordance with the teachings of U.S. Pub. No. 2011/0087212, entitled "Surgical Generator for Ultrasonic and Electrosurgical Devices," published April 14, 2011. While surgical instrument (50) is described herein as an ultrasonic surgical instrument, it should be understood that teachings herein may be readily applied to a variety of surgical instruments, including but not limited to endocutters, graspers, cutters, staplers, clip appliers, access devices, drug/gene therapy delivery devices, and energy delivery devices using ultrasound, RF, laser, etc., and/or any combination thereof as will be apparent to one of ordinary skill in the art in view of the teachings herein. Moreover, while the present example will be described in reference to a cable-connected surgical instrument (50), it should be understood that surgical instrument (50) may be adapted for cordless operation, such as that disclosed in U.S. Pat. Pub. No. 2009/0143797, entitled "Cordless Hand-held Ultrasonic Cautery Cutting Device," published June 4, 2009 . For instance, surgical device (50) may include an integral and portable power source such as a battery, etc. Furthermore, surgical device (50) may also be used, or adapted for use, in robotic-assisted surgery settings such as that disclosed in U.S. Pat. No. 6,783,524, entitled "Robotic Surgical Tool with Ultrasound Cauterizing and Cutting Instrument," issued August 31, 2004.

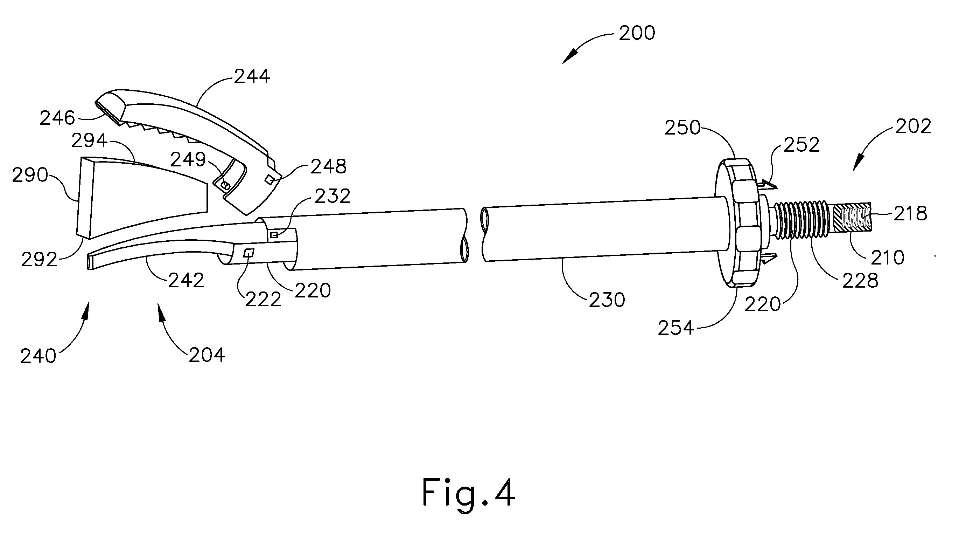

Surgical instrument (50) of the present example includes a multi-piece handle assembly (60), an elongated transmission assembly (70), and a transducer (100). Transmission assembly (70) is coupled to multi-piece handle assembly (60) at a proximal end of transmission assembly (70) and extends distally from multi-piece handle assembly (60). In the present example, transmission assembly (70) is configured as an elongated, thin tubular assembly for endoscopic use, but it should be understood that transmission assembly (70) may alternatively be a short assembly, such as those disclosed in U.S. Pat. Pub. No. 2007/0282333, entitled "Ultrasonic Waveguide and Blade," published December 6, 2007, and U.S. Pat. Pub. No. 2008/0200940, entitled "Ultrasonic Device for Cutting and Coagulating," published August 21, 2008. Transmission assembly (70) of the present example comprises an outer sheath (72), an inner tubular actuating member (not shown), a waveguide (not shown), and an end effector (80) located on the distal end of transmission assembly (70). In the present example, end effector (80) comprises a blade (82) that is mechanically and acoustically coupled to the waveguide, a clamp arm (84) operable to pivot at the proximal end of transmission assembly (70), and a clamp pad (86) coupled to clamp arm (84). Exemplary versions of end effector (80) and transmission assembly (70) will be discussed in greater detail below in reference to the example shown in FIG. 4. Clamp arm (84) and associated features may be constructed and operable in accordance with at least some of the teachings of U.S. Pat. No. 5,980,510, entitled "Ultrasonic Clamp Coagulator Apparatus Having Improved Clamp Arm Pivot Mount," issued November 9, 1999.

In some versions, transducer (100) comprises a plurality of piezoelectric elements (not shown) that are compressed between first resonator (not shown) and second resonator (not shown) to form a stack of piezoelectric elements. The piezoelectric elements may be fabricated from any suitable material, for example, lead zirconate-titanate, lead meta-niobate, lead titanate, and/or any suitable piezoelectric crystal material, for example. Transducer (100) further comprises electrodes, including at least one positive electrode and at least one negative electrode that are configured to create a voltage potential across the one or more piezoelectric elements, such that the piezoelectric elements convert the electrical power into ultrasonic vibrations. The ultrasonic vibrations are transmitted to blade (82) via the waveguide in transmission assembly (70).

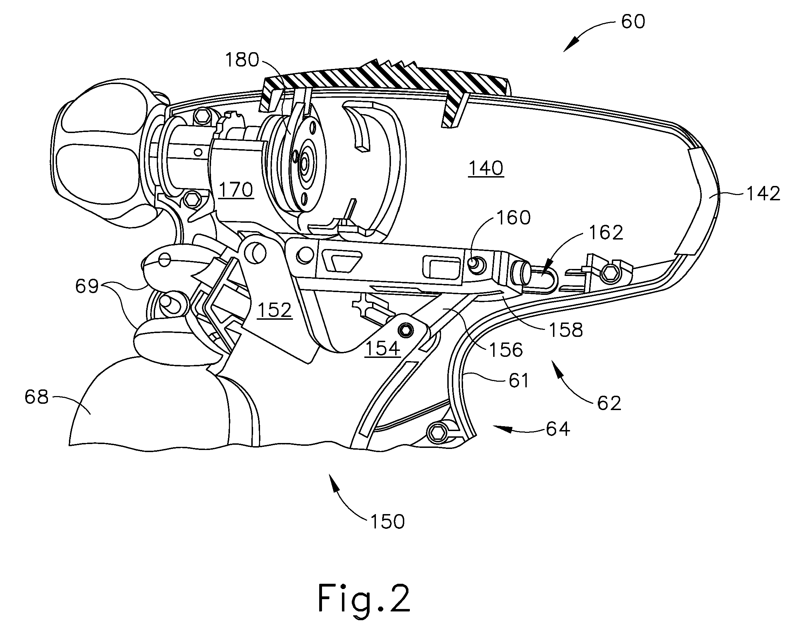

Multi-piece handle assembly (60) of the present example comprises a mating housing portion (62) and a lower portion (64). Mating housing portion (62) is configured to receive transducer (100) at a proximal end of mating housing portion (62) and to receive the proximal end of transmission assembly (70) at a distal end of mating housing portion (62). A rotation knob (66) is shown in the present example to rotate transmission assembly (70) and transducer (100), but it should be understood that rotation knob (66) is merely optional. Mating housing portion (62) will be discussed in greater detail below in reference to FIG. 2. Lower portion (64) of multi-piece handle assembly (60) shown in FIG. 1 includes a trigger (68) and is configured to be grasped by a user using a single hand. One merely exemplary alternative version for lower portion (64) is depicted in FIG. 1 of U.S. Pat. Pub. No. 2011/0015660, entitled "Rotating Transducer Mount for Ultrasonic Surgical Instruments," published January 20, 2011. Toggle buttons (69), shown in FIG. 2 of the present disclosure, are located on a distal surface buttons (69), shown in FIG. 2 of the present disclosure, are located on a distal surface of lower portion (64) and are operable to selectively activate transducer (100) at different operational levels using generator (20). For instance, a first toggle button (69) may activate transducer (100) at a maximum energy level while a second toggle button (69) may activate transducer (100) at a minimum, non-zero energy level. Of course, toggle buttons (69) may be configured for energy levels other than a maximum and/or minimum energy level as will be apparent to one of ordinary skill in the art in view of the teachings herein. Moreover, the toggle buttons may be located anywhere else on multi-piece handle assembly (60), on transducer (100), and/or remote from surgical instrument (50), and any number of toggle buttons may be provided. While multi-piece handle assembly (60) has been described in reference to two distinct portions (62, 64), it should be understood that multi-piece handle assembly (60) may be a unitary assembly with both portions (62, 64) combined. Multi-piece handle assembly (60) may alternatively be divided into multiple discrete components, such as a separate trigger portion (operable either by a user's hand or foot) and a separate mating housing portion (62). Such a trigger portion may be operable to activate transducer (100) and may be remote from mating housing portion (62). Multi-piece handle assembly (60) may be constructed from a durable plastic (such as polycarbonate or a liquid crystal polymer), ceramics, metals, and/or any other suitable material as will be apparent to one of ordinary skill in the art in view of the teachings herein. Other configurations for multi-piece handle assembly (60) will also be apparent to those of ordinary skill in the art in view of the teachings herein. By way of example only, surgical instrument (50) may be constructed in accordance with at least some of the teachings of U.S. Pat. Pub. No. 2006/0079874; U.S. Pat. Pub. No. 2007/0191713; U.S. Pat. Pub. No. 2007/0282333; U.S. Pat. Pub. No. 2008/0200940; U.S. Pat. Pub. No. 2011/0015660; U.S. Pat. No. 6,500,176; U.S. Pat. Pub. No. 2011/0087218; and/or U.S. Pat. Pub. No. 2009/0143797.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### II. Exemplary Coupling Assemblies for Ultrasonic Surgical Instrument

In some instances it may be useful to detach transmission assembly (70) from multi-piece handle assembly (60) and transducer (100). For instance, a detachable transmission assembly (70) may permit the reuse of multi-piece handle assembly (60) with multiple transmission assemblies (70) having various end effectors (80). By way of example only, the various end effectors (80) may have different sized and/or shaped blades (82) or the various end effectors (80) may have entirely different functions, such as RF end effectors, stapling end effectors, cutting end effectors, etc. Furthermore, a single multi-piece handle assembly (60) may be reused for different operations by a user by removing a dirty transmission assembly (70), optionally cleaning multi-piece handle assembly (60), and coupling a new transmission assembly (70) to multi-piece handle assembly (60) for a new operation. Accordingly, configuring multi-piece handle assembly (60) to couple with a variety of transmission assemblies (70) may be preferable for some users of surgical instrument (50).

### A. Exemplary Multi-Piece Handle Assembly

FIG. 2 shows a partial side view of multi-piece handle assembly (60) with a portion of a cover (61) removed to show the internal components contained within mating housing portion (62) and a section of lower portion (64). As described above, lower portion (64) includes a pivotable trigger (68) and a pair of toggle buttons (69). Trigger (68) of the present example is pivotable from a distal, open position to a proximal, closed position. A trigger assembly (150) is coupled to trigger (68) and is pivotally supported within multi-piece handle assembly (60). Trigger assembly (150) of the present example comprises a pivotable attachment arm (152) that may be pivoted about a pin (not shown), a trigger arm (154), an intermediate link (156), and an actuation arm (158). Actuation arm (158) is coupled to a trigger yoke (170) at the distal end of actuation arm (158). Actuation arm (158) comprises one or more mounting pins (160) extending outwardly from actuation arm (158) and pins (160) are sized to be slidably received in corresponding elongated channel (162) formed in cover (61). Accordingly, when trigger (68) is pivoted proximally from the open position to the closed position attachment arm (152) and trigger arm (154) pivot within multi-piece handle assembly (60). Intermediate link (156) coupled to trigger arm (154) transfers this pivoting motion from trigger arm (154) to actuation arm (158) to slidably translate actuation arm (158) proximally via pins (160) within channel (162). Trigger yoke (170), which is coupled to actuation arm (158), is translated proximally as well. In the present example, trigger yoke (170) is coupled to a force-limiting mechanism (180), which is further coupled to transmission assembly (70) as will be described in more detail below, to operate inner tubular actuating member (74). A cavity (140), shown in FIG. 2, is configured to receive transducer (100) therein from a transducer aperture (142) formed in cover (61). Cavity (140) is configured to receive at least a portion of transducer (100) therein such that transducer (100) and transmission assembly (70) may be coupled together. Still other configurations for multi-piece handle assembly (60) will be apparent to one of ordinary skill in the art in view of the teachings herein.

### B. Exemplary Transducer

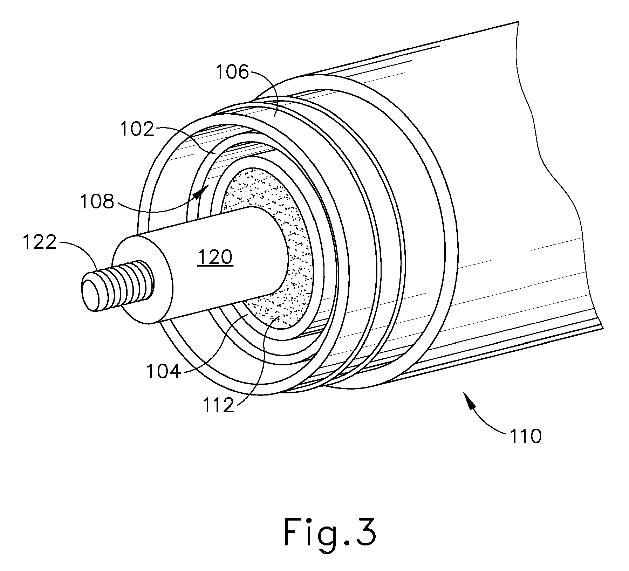

As shown in FIG. 3, transducer (100) of the present example is a tubular component that is coupled to generator (20) via cable (30), though it should be understood that transducer (100) may instead be a cordless transducer. For instance, transducer (100) may instead receive power from a power source that is contained within handle assembly (60), in accordance with the teachings of various references cited herein or otherwise. In the present example, transducer (100) includes a first conductive ring (102) and a second conductive ring (104), which are disposed within a body (110) of transducer (100). In the present example, first conductive ring (102) comprises a ring member having one or more electrical contacts that are disposed on the ring member and that are configured to electrically couple first conductive ring (102) to a power source. First conductive ring (102) is disposed between body (110) and a horn (120) extending distally from body (110). Horn (120) comprises distal horn threads (122) such that horn (120) is coupleable to waveguide (210), as will be discussed below in reference to FIG. 4. First conductive ring (102) of the present example is coaxial with and adjacent to a flange (106). Flange (106) of the present example is configured to further mechanically couple transducer (100) within multi-piece handle assembly (60). A transducer cavity (108) is disposed between first conductive ring (102) and a second conductive ring (104) such that first conductive ring (102) is electrically isolated from second conductive ring (104) and/or other conductive components of transducer (100). First conductive ring (102) is located on a non-conductive platform extending distally from body (110). First conductive ring (102) is electrically coupled to cable (30), shown in FIG. 1, by one or more electrical wires or conductive etchings (not shown) within body (110). Such electrical coupling of first conductive ring (102) to cable (30) may include a slip ring to facilitate free rotation of transducer (100) relative to cable (30).

Second conductive ring (104) of transducer (100) similarly comprises a ring member that is disposed between body (110) and horn (120). Second conductive ring (104) is disposed between first conductive ring (102) and horn (120). As is shown in FIG. 3, first and second conductive rings (102, 104) are coaxial members. Second conductive ring (104) is likewise electrically isolated from first conductive ring (102) and other conductive components of transducer (100). Similar to first conductive ring (102), second conductive ring (104) extends from the non-conductive platform. One or more washer-shaped spacers (112) may be disposed between second conductive ring (104) and horn (120) to isolate the vibrations transmitted through horn (120) from the other components of transducer (100). Second conductive ring (104) is also electrically coupled to cable (30), shown in FIG. 1, by one or more electrical wires or conductive etchings (not shown) within body (110). Such electrical coupling of second conductive ring (104) to cable (30) may also include a slip ring to facilitate free rotation of transducer (100) relative to cable (30). One merely exemplary suitable ultrasonic transducer (100) is Model No. HP054, sold by Ethicon Endo-Surgery, Inc. of Cincinnati, Ohio, though it should be understood that any other suitable transducer may be used.

As shown in the present example, the distal end of transducer (100) threadably couples to the proximal end of a transmission assembly via horn (120). The distal end of transducer (100) also interfaces with one or more electrical connections (not shown) via first and second conductive rings (102, 104) to electrically couple transducer (100) to toggle buttons (69) to provide a user with finger-activated controls for activating transducer (100) while using surgical instrument (50). The interface between the one or more electrical connections and the first and second conductive rings (102, 104) may include a slip ring connection to permit free rotation of transducer (100) relative to multi-piece handle assembly (60). Still other configurations for transducer (100) will be apparent to one of ordinary skill in the art in view of the teachings herein. For instance, first and second conductive rings (102, 104) may be omitted from the distal end of transducer (100) and the electrical coupling of transducer (100) to toggle buttons (69) may be accomplished by alternative structures, such as conductors at the proximal end of transducer (100), conductors located along the side of body (110) of transducer (100), directly from cable (30), and/or otherwise. When transducer (100) of the present example is activated via a toggle button (69), transducer (100) is operable to create mechanical energy in the form of linear oscillations or vibrations, at an ultrasonic frequency (such as 55.5 kHz). When transducer (100) is coupled to transmission assembly (70) via horn (120), these mechanical oscillations are transmitted through the internal waveguide of transmission assembly (70) to end effector (80). In the present example, with blade (82) being coupled to the waveguide, blade (82) thereby oscillates at the ultrasonic frequency. Thus, when tissue is secured between blade (82) and clamp arm (84), the ultrasonic oscillation of blade (82) may simultaneously sever the tissue and denature the proteins in adjacent tissue cells, thereby providing a coagulative effect with relatively little thermal spread. An electrical current may also be provided through blade (82) and clamp arm (84) to also cauterize the tissue. While some configurations for transmission assembly (70) and transducer (100) have been described, still other suitable configurations for transmission assembly (70) and transducer (100) will be apparent to one of ordinary skill in the art in view of the teachings herein.

### C. Exemplary Transmission Assembly for Threaded Attachment

As noted previously, in some instances it may be useful to detach transmission assembly (70) from multi-piece handle assembly (60) and transducer (100). Merely exemplary instances include the use of multi-piece handle assembly (60) with multiple transmission assemblies (70) having different sized and/or shaped blades (82), use with various end effectors (80) with entirely different functions and/or modalities (e.g., RF end effectors, stapling end effectors, cutting end effectors, etc.), or for reuse of a single multi-piece handle assembly (60) for multiple operations by a user. Accordingly, a version permitting the user to swap transmission assemblies (70) with multi-piece handle assembly (60) may be useful.

One merely exemplary transmission assembly (200) is shown in FIG. 4 having a proximal end (202), a distal end (204), a waveguide (210), an inner tubular actuating member (220), an outer sheath (230), and an end effector (240) at the distal end of transmission assembly (200). In the present example, waveguide (210), inner tubular actuating member (220), and outer sheath (230) are coaxial members with waveguide (230) in the center, inner actuating member (220) disposed about waveguide (210), and outer sheath (230) disposed about inner actuating member (220).

Referring to distal end (204) of transmission assembly (200) first, end effector (240) comprises a blade (242), a clamp arm (244), and one or more optional clamp pads (246). In the present example, blade (242) is coupled to waveguide (210) such that the mechanical vibrations transmitted to waveguide (210) from transducer (100) are also transmitted to blade (242). Merely exemplary couplings for blade (242) to waveguide (210) include welding blade (242) to waveguide (210), integrally forming blade (242) with waveguide (210), mechanically or chemically coupling blade (242) to waveguide (210), and/or any other suitable configuration as will be apparent to one of ordinary skill in the art in view of the teachings herein. In some versions, blade (242) is a curved blade, such as blade (242) shown in FIG. 4; and in some versions blade (242) may be a straight blade. Furthermore, blade (242) may have a variety of shapes and sizes. In the present example, blade (242) is a tapered rectangular blade, though it should be understood that blade (242) may be cylindrical, triangular, hemi-cylindrical, square, hooked, and/or any other shape for blade (242). Furthermore, additional features may be added to blade (242), including spherical tips, hooked tips, square tips, serrated edging, and/or any other additional features. Still other configurations for blade (242) will be apparent to those of ordinary skill in the art in view of the teachings herein.

Clamp arm (244) of the present example is a curved member that corresponds to the curvature of blade (242). Clamp arm (244) may optionally include clamp pads (246) to grip or secure tissue against blade (242). Such clamp pads may be configured in accordance with at least some of the teachings of U.S. Pat. Pub. No. 2006/0079874, entitled "Tissue Pad Use with an Ultrasonic Surgical Instrument," published April 13, 2006. Pivotal movement of clamp arm (244) with respect to blade (242) is accomplished by a first pair of pivot points (248) on clamp arm (244) that pivotally couple to outer sheath (230) and a second set of pivot points (249) on clamp arm (244) that pivotally couple to inner tubular actuating member (220). In the present example, outer sheath (230) is coupleable to multi-piece handle assembly (60) through a rotation knob (250), thereby grounding outer sheath (230). First set of pivot points (248) of clamp arm (244) are pivotally connected to outer sheath (230) via corresponding through holes (232) on outer sheath (230). In some versions, first set of pivot points (248) comprise through holes and a securing pin or rivet may be inserted through first set of pivot points (248) and through through holes (232) to secure clamp arm (244) to outer sheath (230). The pin in this version may be laser welded to clamp arm (244) or the pin may be laser welded to outer sheath (230). Of course through holes (232) may instead be outwardly extending pins and first set of pivot points (248) may be through holes. Still other configurations for first set of pivot points (248) and through holes (232) will be apparent to one of ordinary skill in the art in view of the teachings herein.

Second set of pivot points (249) of clamp arm (244) are pivotally connected to inner tubular actuating member (220) via corresponding through holes (222) on inner tubular actuating member (220). In some versions, second set of pivot points (249) comprise through holes and a securing pin or rivet may be inserted through second set of pivot points (249) and through through holes (222) to secure clamp arm (244) to inner tubular actuating member (220). The pin in this version may be laser welded to clamp arm (244) or the pin may be laser welded to inner tubular actuating member (220). Of course through holes (222) may instead be outwardly extending pins and second set of pivot points (249) may be through holes. Still other pivotable configurations for second set of pivot points (249) and through holes (222) will be apparent to one of ordinary skill in the art in view of the teachings herein.

With clamp arm (244) so secured to outer sheath (230) and inner tubular actuating member (220), clamp arm (244) is pivotable when inner tubular actuating member (220) translates longitudinally. In the present example, inner tubular actuating member (220) is translatable relative to the longitudinal axis of outer sheath (230) and is coupled to force-limiting mechanism (180) within multi-piece handle assembly (60). Thus, when force-limiting mechanism (180) translates via trigger (68) and trigger assembly (150), clamp arm (244) is pivotable from an open position to a closed position. It should be understood that, as with other components referred to herein, clamp arm (84, 244) is merely optional. Likewise, trigger (68) and trigger assembly (150) and the components described herein for pivoting clamp arm (84, 244) are also merely optional. Thus, some versions of end effector (80, 240) may simply consist of a blade (82, 842) and/or other features.

As shown in FIG. 4, a spacer (290) is insertable between clamp arm (244) and blade (242) to maintain clamp arm (244) in the open position. Spacer (290) has a flat bottom surface (292) and an angled top surface (294) in this example. Top surface (294) is set at an angle to maintain clamp arm (244) in the open position relative to blade (242) when bottom surface (292) abuts blade (242). In some versions, bottom surface (292) may be configured to snap or clip onto blade (242) to secure spacer (290) relative to blade (242). Alternatively, a recess may be provided in spacer (290) such that spacer (290) may be slid onto blade (242). Further still, an adhesive may be applied to bottom surface (292) and/or top surface (294) to also secure spacer (290). Thus, when spacer (290) is inserted between clamp arm (244) and blade (242), clamp arm (244) is prevented from pivoting to a closed position. This may permit a user to couple transmission assembly (200) to multi-piece handle assembly (60) while maintaining both clamp arm (244) and trigger (68) in their respective open positions. Alternatively, a user may couple transmission assembly (200) to multi-piece handle assembly (60) without the use of spacer (290). For example, the user may couple different components of transmission assembly (200) with different components of handle assembly (60) at different times, such as in the manner described below or otherwise.

Referring now to proximal end (202) of transmission assembly (200), a rotation knob (250) couples outer sheath (230) to multi-piece handle assembly (60). In the present example, rotation knob (250) comprises an inner ring portion (not shown) having one or more connectors (252) extending proximally therefrom, an outer ring (254), and a pin (not shown) extending through outer ring (254), outer sheath (230), inner tubular actuating member (220), and waveguide (210). Accordingly, when outer ring (254) of rotation knob (250) is rotated, waveguide (210), inner tubular actuating member (220), and outer sheath (230) also rotate. Inner ring portion and outer ring (254) of the present example are complementary bearing components such that outer ring (254) is rotatable relative to inner ring portion. It should be understood that the pin does not extend though inner ring portion. As previously noted, inner ring portion includes connectors (252). In the present example connectors (252) are shown as snap-fit connectors, though other suitable connecting features, such as threading, adhesives, pins, clips, snaps, and/or other connectors may be used as will be apparent to one of ordinary skill in the art in view of the teachings herein. When transmission assembly (200) is assembled with multi-piece handle assembly (60) and transducer (100), as will be discussed below, connectors (252) of the present example insert into one or more recesses (not shown) and couple rotation knob (250) to cover (61) of multi-piece handle assembly (60). A release mechanism, such as a push button (not shown) on multi-piece handle assembly (60) or on rotation knob (250) may be provided to decouple connectors (252) from cover (61) when transmission assembly (200) is to be removed. Alternatively, connectors (252) may be designed to break-away when transmission assembly (200) is decoupled. Further still, if threading is used, inner portion of rotation knob (250) may be rotated to decouple from multi-piece handle assembly (60). Still other suitable configurations for rotation knob (250) will be apparent to one of ordinary skill in the art in view of the teachings herein.

Still referring to proximal end (202) of transmission assembly (200), external threads (228) are included at the proximal end of inner tubular actuating member (220) as shown in FIG. 4. External threads (228) screw into complementary threads (not shown) of force-limiting mechanism (180), which is in turn driven by trigger assembly (150). Additionally, a recess having internal threading (218) is included at the proximal end of waveguide (210) as shown in FIG. 4. Internal threading (218) screws onto horn threads (122) to mechanically and acoustically couple waveguide (210) to transducer (100). Of course other suitable configurations for transmission assembly (200) will be apparent to one or ordinary skill in the art in view of the teachings herein. Similarly, various other suitable ways in which transmission assembly (200) may be coupled with handle assembly (60) will be apparent to those of ordinary skill in the art in view of the teachings herein.

### III. Exemplary Incorporation of Suction and/or Irrigation in Ultrasonic Surgical System

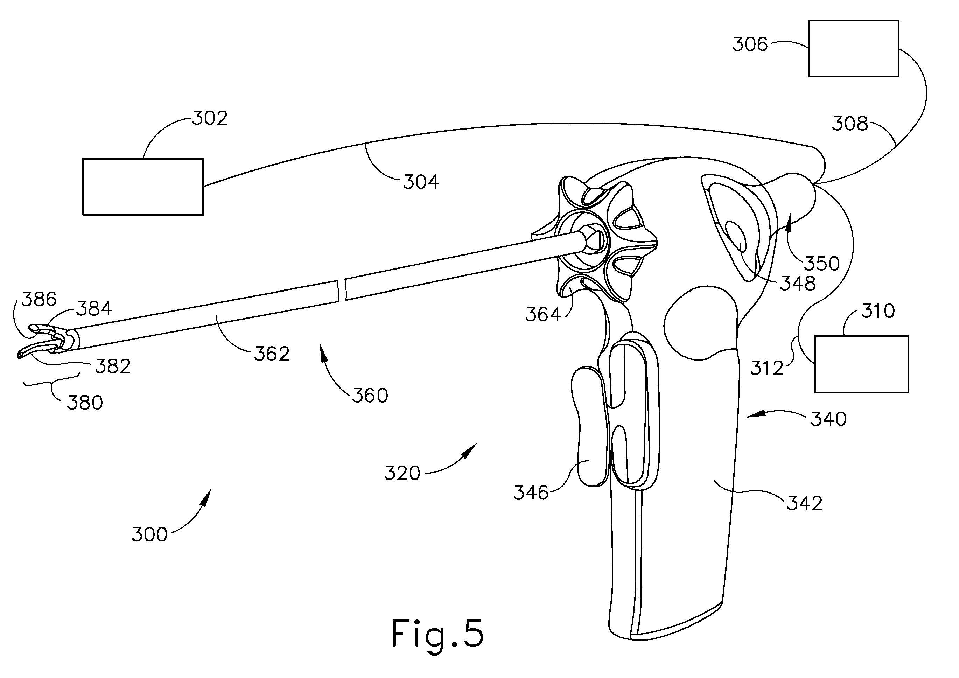

FIG. 5 shows an exemplary ultrasonic surgical system (300) comprising a generator (302), a vacuum source (306), a fluid source (310), and an ultrasonic surgical instrument (320). Generator (302) is coupled with instrument (320) via a cable (304) in the present example, though it should be understood that generator (302) and/or some other power source may be integrated into instrument (320). Generator (302) may be constructed and operable similar to generator (20) described above. Vacuum source (306) is coupled with instrument (320) via a conduit (308) and is operable to provide suction at an end effector (380) of instrument (320) as will be described in greater detail below. Like generator (302), vacuum source (306) may be integrated into instrument (320) in some versions. It should also be understood that vacuum source (306) is merely optional and may be omitted in some versions. Fluid source (310) is coupled with instrument (320) via a conduit (312) and is operable to provide fluid for dispensation through end effector (380) as will be described in greater detail below. It should be understood that fluid source (310) may also be integrated into instrument (320) or may simply be omitted in some versions. It should also be understood that fluid source (310) may be configured to dispense one or more fluids, including but not limited to an irrigation fluid (e.g., saline); any of the various medical fluids described in U.S. Pub. No. 2011/0152759, entitled "Use of Biomarkers and Therapeutic Agents with Surgical Devices," published June 23, 2011, any of the various medical fluids described in U.S. Pat. App. No. 12/779,400, entitled "Multi-Chamber Therapeutic Cell Applicator Instrument," filed May 13, 2010, and/or any other suitable type of fluid. It should be understood that system (300) may be readily used in accordance with at least some of the teachings of any reference cited herein and/or in any other suitable fashion. It should also be understood that instrument (320) may deliver two or more of suction, fluid, and/or ultrasonic energy through end effector (380) substantially simultaneously.

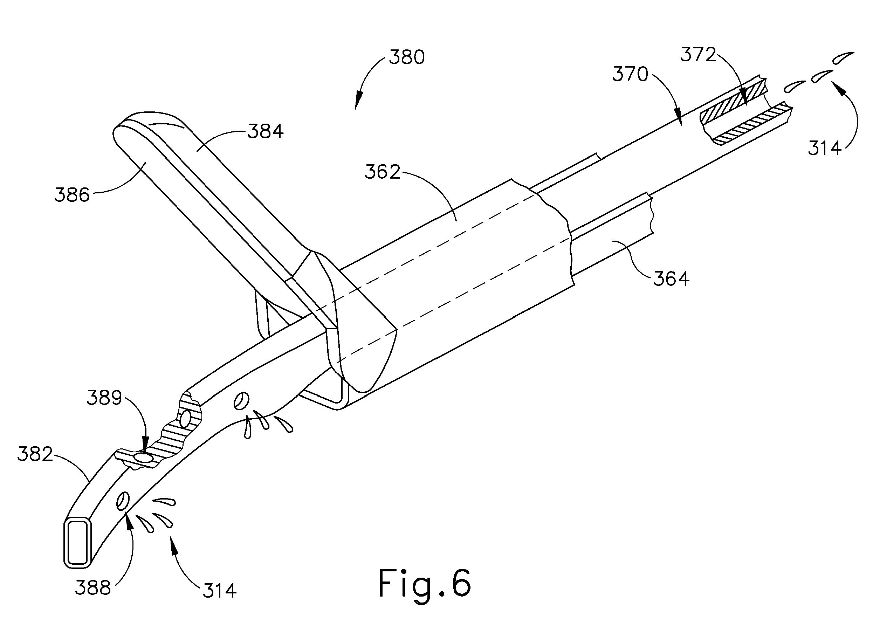

Instrument (320) of the present example is substantially similar to instrument (10) described above in several respects. For instance, instrument (320) includes a handle assembly (340) with a grip (342), a trigger (346), and a button (348). Trigger (346) is operable in a manner similar to trigger (68) described above. Button (348) is operable in a manner similar to buttons (69) described above. A transmission assembly (360) extends distally from handle assembly (340) and is rotatable relative to handle assembly (340) via a knob (364). End effector (380) is at the distal end of transmission assembly (360) and is operable in a manner similar to end effector (380) described above. FIG. 6 shows end effector (380) in greater detail. As shown, end effector (380) includes a harmonic blade (382), which is in acoustic communication with a waveguide (370) that extends through transmission assembly (360). End effector (380) also includes a pivotable clamp arm (384) with a clamp pad (386). Clamp arm (384) is coupled with an actuating member (364), which is translatable within outer sheath (362) of transmission assembly (360) like actuating member (220) to selectively pivot clamp arm (384) toward and away from harmonic blade (382). Like blade (82), blade (382) of this example may be selectively activated at ultrasonic frequencies from a transducer (350), which will be described in greater detail below.

Unlike blade (82), blade (382) of the present example includes openings (388) that are in communication with a hollow interior (389) of blade (382). Waveguide (370) of this example defines a lumen (372) that is in fluid communication with the hollow interior (389) of blade (382). Lumen (372) of waveguide (370) is also in fluid communication with vacuum source (306) and/or fluid source (310) as will be described in greater detail below. Thus, suction and/or fluid (314) may be communicated through lumen (372), through hollow interior (389), and through openings (388) to a surgical site. Suction may be provided through openings (388) to evacuate vapor, smoke, blood, other bodily fluid, etc. from the surgical site. Fluid (314) may be provided through openings (388) to irrigate the surgical site, to treat tissue at the surgical site, and/or for any other suitable purpose(s). While openings (388) of the present example are presented on lateral sides of blade (382), it should be understood that one or more openings (388) may be positioned at the distal end of blade (382) and/or at any other suitable location(s), in addition to or in lieu of being positioned on lateral sides of blade (382).

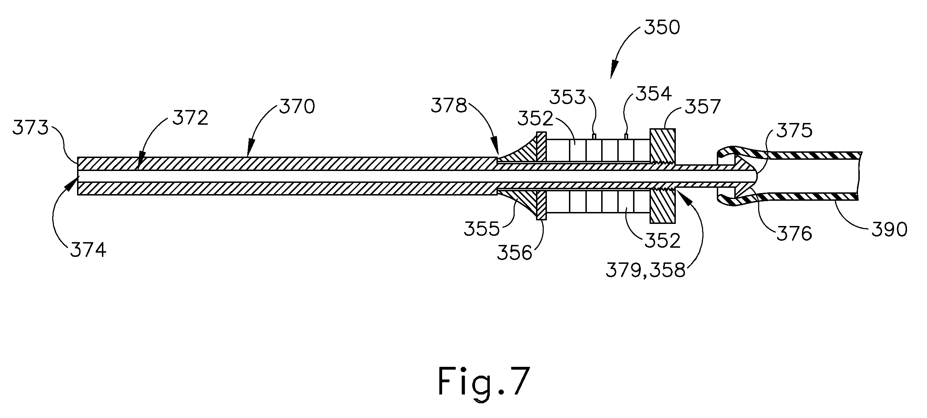

FIG. 7 shows waveguide (370) and transducer (350) in greater detail. As can be seen, lumen (372) of waveguide (370) extends from the proximal end (375) of waveguide (370) to an opening (374) formed at the distal end (373) of waveguide (370). Blade (382) of the present example is secured to distal end (373) of waveguide (370) through a threaded coupling, though it should be understood that blade (382) may be coupled with waveguide (370) in any other suitable fashion. It should also be understood that blade (382) and waveguide (370) may constitute a unitary monolithic structure, such that blade (382) and waveguide (370) are formed as a homogenous continuum of material in some versions. A conduit (390) is coupled with proximal end (375) of waveguide (370), which includes a barb (376) to assist in retaining conduit (390). In some versions, a collar (not shown) is secured about the exterior of conduit (390) in the region of barb (376) to further secure the coupling between conduit (390) and waveguide (370). Other suitable ways in which conduit (390) may be coupled with waveguide (370) will be apparent to those of ordinary skill in the art in view of the teachings herein.

Conduit (390) may comprise an elastomeric material and/or any suitable material having any other suitable properties. Conduit (390) of the present example is in fluid communication with conduits (308, 312). In versions lacking vacuum source (306), conduit (390) may simply couple lumen (372) of waveguide (370) directly to fluid source (310). Similarly, in versions lacking fluid source (310), conduit (390) may simply couple lumen (372) of waveguide (370) directly to vacuum source (306). In versions where both vacuum source (306) and fluid source (310) are present, a manifold (not shown) may couple conduit (390) with conduits (308, 312). One or more valves and/or other features may be used to selectively prevent one conduit (308, 312) from communicating with conduit (390) when the other conduit (308, 312) is communicating with conduit (390). As yet another merely illustrative variation, waveguide (370) may include a pair of lumens (372) that are fluidly isolated relative to each other, with each lumen (372) being in communication with a respective conduit (308, 312). Blade (380) may include one or more openings (388) that are dedicated to each of such lumens (372). Such lumens (372) may be arranged coaxially with each other, parallel yet laterally offset relative to each other, and/or otherwise. Still other suitable ways in which vacuum source (306) and/or fluid source (310) may be in fluid communication with waveguide (370) will be apparent to those of ordinary skill in the art in view of the teachings herein.

Transducer (350) of the present example comprises a stack of piezoelectric elements (352) that are configured and operable in accordance with the piezoelectric elements described above. Piezoelectric elements (352) include a pair of contacts (353, 354) that are in electrical communication with generator (302), such that generator (302) may be used to selectively activate transducer (350). In some versions, contacts (353, 354) communicate with generator (302) via a slip ring assembly (not shown), such that transducer (350), waveguide (370), transmission assembly (360), and end effector (380) are collectively rotatable relative to handle assembly (340) without causing wires to twist and bind, etc. The fluid coupling of conduit (390) may also permit such rotation.

Waveguide (370) extends fully through a bore defined by transducer (350) in this example, such that transducer (350) is positioned coaxially about waveguide (370), such that the distal end (373) of waveguide (370) is distal to the distal end of transducer (350), and such that the proximal end (375) of waveguide (370) is proximal to the proximal end of transducer (350). As noted above, waveguide (370) is formed as a tube. By way of example only, waveguide (370) may be gun-drilled to form lumen (372). As another merely illustrative example, waveguide (370) may be drawn. Other suitable ways in which waveguide (370) may be formed will be apparent to those of ordinary skill in the art in view of the teachings herein.

A horn (355) is positioned coaxially about waveguide (370) and longitudinally distal to transducer (350). Horn (355) engages a shoulder (378) formed in waveguide (370). In some versions, shoulder (378) is located at an antinode of the ultrasonic vibrational wave communicated through waveguide (370), though it should be understood that shoulder (378) may be provided at any other suitable location. A washer (356) is interposed between transducer (350) and horn (355). A compression nut (357) is positioned coaxially about waveguide (370) and longitudinally proximal to transducer (350). Compression nut (357) includes internal threading (379) that complements external threading (358) of waveguide (370). Thus, compression nut (357) may be rotated relative to waveguide (370) to drive transducer (350) into washer (356), washer (356) into horn (355), and horn (355) into shoulder (378). Horn (355) thereby transmits ultrasonic vibrations generated by transducer (350) to waveguide (370).

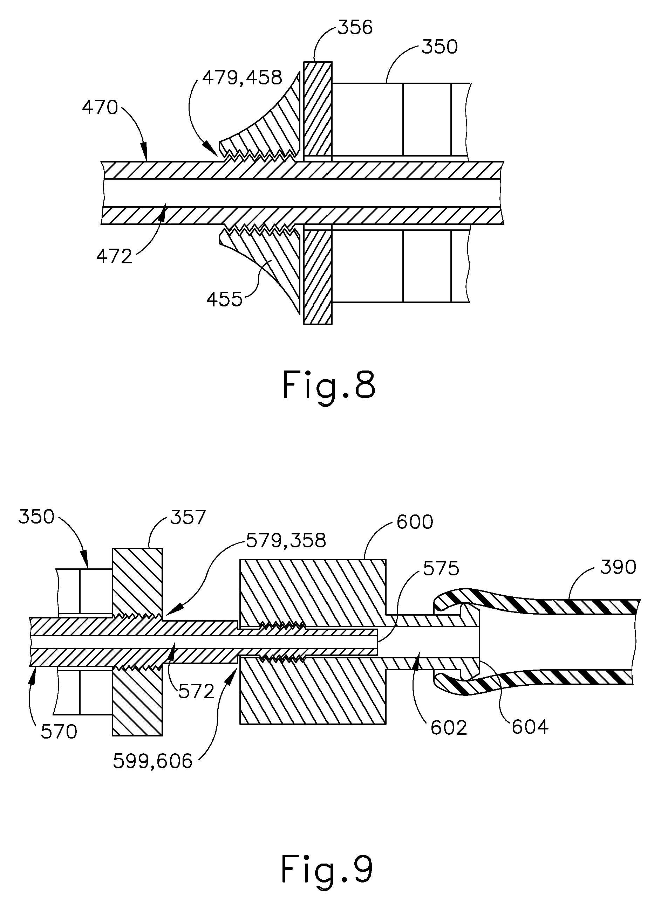

FIG. 8 shows an exemplary alternative coupling between a horn and a hollow waveguide. In this example, horn (455) is substantially similar to horn (355) except that horn (455) of this example includes internal threading (458). Waveguide (470) of this example is substantially similar to waveguide (370) except that waveguide (470) of this example includes external threading (479) instead of including shoulder (378). Thus, horn (455) is secured to waveguide (470) through engagement of threading (458, 479). Waveguide (470) of this example still includes a lumen (472), and transducer (350) is still secured against washer (356) and horn (455) via a compression nut (357) in this example, such that ultrasonic vibrations are still transmitted to waveguide (470) via horn (455).

FIG. 9 shows an exemplary alternative coupling between a waveguide and a fluid conduit. In this example, waveguide (570) is substantially similar to waveguide (370) except that waveguide (570) of this example includes extra external threading (599) and lacks barb (376). The proximal end (575) of waveguide (570) is located within a lumen (602) formed in an acoustic mass (600). Acoustic mass (600) includes internal threading (606) engaging external threading (599) of waveguide (570), thereby securing acoustic mass (600) to waveguide (570).

Conduit (390) is coupled with acoustic mass (600), such that conduit (390) is in fluid communication with lumen (602). Lumen (572) of waveguide (570) is thus in fluid communication with conduit (390) via lumen (602) of acoustic mass (600). The proximal end of acoustic mass (600) includes an outwardly extending flange (604) to help secure conduit (390) to acoustic mass (600). A collar, cuff, and/or other suitable feature may be provided to further secure conduit (390) to acoustic mass (600). Acoustic mass (600) is configured to reduce the vibrational amplitude of waveguide (570) at the point where conduit (390) couples with acoustic mass (600). This reduces the vibrational impedance created by conduit (390).

In the present example, the proximal face of compression nut (357) is located at a longitudinal position associated with an antinode of the ultrasonic vibrational wave communicated through waveguide (570); while the distal face of acoustic mass (600) is located at a longitudinal position associated with a node of the ultrasonic vibrational wave communicated through waveguide (570). In some versions, threading (606, 599) is located at a node of the ultrasonic vibrational wave communicated through waveguide (570). In some other versions, threading (506, 599) is located at an antinode of the ultrasonic vibrational wave communicated through waveguide (570). Alternatively, threading (506, 699) may be located elsewhere. In addition or in the alternative, flange (604) may be located at a node, at an antinode, or at any other suitable location. In versions where threading (599, 606) is located at a node, flange (604) may also be located at a node; or flange (604) may instead be located at an antinode or elsewhere. Likewise, in versions where threading (599, 606) is located at an antinode, flange (604) may also be located at an antinode; or flange (604) may instead be located at a node or elsewhere. Of course, any other suitable positioning may be used.

### IV. Miscellaneous

It should be understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional endoscopic and open surgical instrumentation as well as application in robotic-assisted surgery. For instance, those of ordinary skill in the art will recognize that various teaching herein may be readily combined with various teachings of U.S. Pat. No. 6,783,524, entitled "Robotic Surgical Tool with Ultrasound Cauterizing and Cutting Instrument," published August 31, 2004.

Versions of described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention is defined by the following claims and is understood not to be limited to the details of structure and operation shown and described in the specification and drawings.

## Claims

1. A surgical system (300) comprising:
(a) an ultrasonic surgical instrument (320) comprising:
(i) an ultrasonic transducer (350), the transducer having a distal end and a proximal end, wherein the transducer defines a bore extending from the proximal end to the distal end, wherein the transducer is operable to convert electrical power into ultrasonic vibrations,
(ii) a waveguide (370) disposed in the bore of the transducer, wherein the waveguide has a proximal end located proximal to the proximal end of the transducer, wherein the waveguide has a distal end located distal to the distal end of the transducer, wherein the waveguide defines a lumen extending from the proximal end of the waveguide to the distal end of the waveguide, and
(iii) an end effector (380) in acoustic communication with the waveguide, wherein the waveguide is operable to transmit ultrasonic vibrations from the transducer to the end effector;
(b) a horn (355) coaxially disposed about the waveguide, wherein the horn is distal to the transducer, wherein the horn is configured to be brought in contact with the waveguide;
(c) a compression nut (357) coaxially disposed about the waveguide, wherein the compression nut is proximal to the transducer, wherein the compression nut is operable to urge the transducer toward the horn and thereby urge the horn into engagement with the waveguide so that the horn transmits ultrasonic vibrations generated by the transducer to the waveguide; and
(d) a conduit (390) coupled with the lumen of the waveguide, wherein the conduit is operable to communicate with one or both of a vacuum source or a fluid source to transmit one or both of suction or fluid through the lumen.

2. The surgical system of claim 1, wherein the waveguide consists of a single monolithic component formed as a homogenous continuum of material.

3. The surgical system of claim 1, wherein the end effector is in fluid communication with the lumen, such that the end effector is operable to transmit one or both of suction or fluid from the lumen to a surgical site.

4. The surgical system of claim 1, wherein the end effector comprises a harmonic blade in acoustic communication with the waveguide.

5. The surgical system of claim 4, wherein the harmonic blade comprises one or more openings in fluid communication with the lumen of the waveguide.

6. The surgical system of claim 5, wherein at least one of the one or more openings is positioned on an axis oriented transversely to a longitudinal axis defined by the harmonic blade.

7. The surgical system of claim 1, wherein the proximal end of the waveguide includes a barb, wherein the conduit is secured to the barb.

8. The surgical system of claim 1, wherein the waveguide includes a shoulder, wherein the horn is engaged with the shoulder of the waveguide.

9. The surgical system of claim 1, wherein the horn and the waveguide include complementary threading, wherein the horn is secured to the waveguide through the complementary threading.

10. The surgical system of claim 9, wherein the compression nut and the waveguide include complementary threading, wherein the compression nut is secured to the waveguide through the complementary threading.

11. The surgical system of claim 1, further comprising an acoustic mass secured to the waveguide, wherein the acoustic mass is spaced apart from the transducer.

12. The surgical system of claim 11, , wherein the acoustic mass is located proximal to the transducer.

13. The surgical system of claim 11, wherein the acoustic mass and the waveguide include complementary threading, wherein the acoustic mass is secured to the waveguide through the complementary threading.

14. The surgical system of claim 11, wherein the acoustic mass defines a lumen in fluid communication with the lumen of the waveguide.

15. The surgical system of claim 14, wherein the conduit is coupled with the acoustic mass such that the conduit is in fluid communication with the lumen of the acoustic mass, wherein the acoustic mass includes a conduit retention feature configured to secure the conduit to the acoustic mass.

16. The surgical system of claim 11, wherein the acoustic mass includes a distal face, wherein the distal face is longitudinally positioned at a node associated with the waveguide.

## Patentansprüche

1. Chirurgisches System (300), umfassend:
(a) ein chirurgisches Ultraschallinstrument (320), umfassend:
(i) einen Ultraschallwandler (350), wobei der Wandler ein distales Ende und ein proximales Ende hat, wobei der Wandler eine sich vom proximalen Ende zum distalen Ende erstreckende Bohrung definiert, wobei der Wandler dahingehend betreibbar ist, elektrische Leistung in Ultraschallschwingungen umzuwandeln,
(ii) einen Wellenleiter (370),
der in der Bohrung des Wandlers angeordnet ist, wobei der Wellenleiter ein proximal zum proximalen Ende des Wandlers angeordnetes proximales Ende hat, wobei der Wellenleiter ein distal zum distalen Ende des Wandlers angeordnetes distales Ende hat, wobei der Wellenleiter ein sich vom proximalen Ende des Wellenleiters zum distalen Ende des Wellenleiters erstreckendes Lumen definiert, und
(iii) einen Endeffektor (380)
in akustischer Kommunikation mit dem Wellenleiter, wobei der Wellenleiter dahingehend betreibbar ist, Ultra-schallschwingungen vom Wandler zum Endeffektor zu übertragen,
(b) ein Horn (355),
das koaxial um den Wellenleiter angeordnet ist, wobei das Horn distal zum Wandler angeordnet ist, wobei das Horn dazu konfiguriert ist, mit dem Wellenleiter in Kontakt gebracht zu werden,
(c) eine Druckmutter (357),
die koaxial um den Wellenleiter angeordnet ist, wobei die Druckmutter proximal zum Wandler angeordnet ist, wobei die Druckmutter dahingehend betreibbar ist, den Wandler zum Horn hin zu drängen und dadurch das Horn in Eingriff mit dem Wellenleiter zu drängen, so dass das Horn vom Wandler erzeugte Ultra-schallschwingungen an den Wellenleiter überträgt, und
(d) eine Leitung (390),
die mit dem Lumen des Wellenleiters gekoppelt ist, wobei die Leitung dahingehend betreibbar ist, mit einer Vakuumquelle oder einer Fluidquelle oder beiden in Verbindung zu stehen, um Saugung oder Fluid oder beides durch das Lumen zu übertragen.

2. Chirurgisches System nach Anspruch 1, wobei der Wellenleiter aus einer einzelnen monolithischen Komponente besteht, die als ein homogenes Materialkontinuum ausgebildet ist.

3. Chirurgisches System nach Anspruch 1, wobei der Endeffektor mit dem Lumen in Fluidverbindung steht, so dass der Endeffektor dahingehend betreibbar ist, Saugung oder Fluid oder beides von dem Lumen an einen operativen Situs zu übertragen.

4. Chirurgisches System nach Anspruch 1, wobei der Endeffektor ein harmonisches Skalpell in akustischer Kommunikation mit dem Wellenleiter umfasst.

5. Chirurgisches System nach Anspruch 4, wobei das harmonische Skalpell eine oder mehrere Öffnungen in Fluidverbindung mit dem Lumen des Wellenleiters umfasst.

6. Chirurgisches System nach Anspruch 5, wobei mindestens eine der einen oder mehreren Öffnungen auf einer quer zu einer durch das harmonische Skalpell definierten Längsachse ausgerichteten Achse positioniert ist.

7. Chirurgisches System nach Anspruch 1, wobei das proximale Ende des Wellenleiters einen Widerhaken aufweist, wobei die Leitung an dem Widerhaken befestigt ist.

8. Chirurgisches System nach Anspruch 1, wobei der Wellenleiter eine Schulter aufweist, wobei das Horn mit der Schulter des Wellenleiters in Eingriff steht.

9. Chirurgisches System nach Anspruch 1, wobei das Horn und der Wellenleiter ein komplementäres Gewinde aufweisen, wobei das Horn durch das komplementäre Gewinde an dem Wellenleiter befestigt ist.

10. Chirurgisches System nach Anspruch 9, wobei die Druckmutter und der Wellenleiter ein komplementäres Gewinde aufweisen, wobei die Druckmutter durch das komplementäre Gewinde an dem Wellenleiter befestigt ist.

11. Chirurgisches System nach Anspruch 1, ferner umfassend eine am Wellenleiter befestigte akustische Masse, wobei die akustische Masse vom Wandler be-abstandet ist.

12. Chirurgisches System nach Anspruch 11, wobei die akustische Masse proximal zum Wandler angeordnet ist.

13. Chirurgisches System nach Anspruch 11, wobei die akustische Masse und der Wellenleiter ein komplementäres Gewinde aufweisen, wobei die akustische Masse durch das komplementäre Gewinde an dem Wellenleiter befestigt ist.

14. Chirurgisches System nach Anspruch 11, wobei die akustische Masse ein Lumen in Fluidverbindung mit dem Lumen des Wellenleiters definiert.

15. Chirurgisches System nach Anspruch 14, wobei die Leitung mit der akustischen Masse gekoppelt ist, so dass die Leitung in Fluidverbindung mit dem Lumen der akustischen Masse ist, wobei die akustische Masse ein Leitungshaltemerkmal aufweist, das dazu konfiguriert ist, die Leitung an der akustischen Masse zu befestigen.

16. Chirurgisches System nach Anspruch 11, wobei die akustische Masse eine distale Fläche aufweist, wobei die distale Fläche in Längsrichtung an einem dem Wellenleiter zugeordneten Knoten positioniert ist.

## Revendications

1. Système (300) chirurgical comportant:
(a) un instrument (320) chirurgical ultrasonore comportant:
(i) un transducteur (350) ultrasonore,
le transducteur présentant une extrémité distale et une extrémité proximale, dans lequel le transducteur définit un alésage qui s'étend de l'extrémité proximale à l'extrémité distale, le transducteur étant fonctionnel pour convertir l'énergie électrique en vibrations ultrasonores,
(ii) un guide d'onde (370)
disposé sur l'alésage du transducteur, dans lequel le guide d'onde présente une extrémité proximale située de manière proximale par rapport à l'extrémité proximale du transducteur, dans lequel le guide d'onde présente une extrémité distale située de manière distale par rapport à l'extrémité distale du transducteur, le guide d'onde définissant un passage qui s'étend de l'extrémité proximale du guide d'onde à une extrémité distale du guide d'onde, et
(iii) un effecteur (380) d'extrémité
en communication acoustique avec le guide d'onde, dans lequel le guide d'onde est fonctionnel pour transmettre des vibrations ultrasonores du transducteur à l'effecteur d'extrémité ;
(b) un avertisseur sonore (355)
disposé coaxialement autour du guide d'onde, dans lequel l'avertisseur sonore est distal par rapport au transducteur, l'avertisseur sonore étant configuré pour être amené en contact avec le guide d'onde;
(c) un écrou (357) de compression
disposé coaxialement autour du guide d'onde, dans lequel l'écrou de compression est proximal par rapport au transducteur, l'écrou de compression étant fonctionnel pour pousser le transducteur vers l'avertisseur sonore et par conséquent pousser l'avertisseur sonore à venir en prise avec le guide d'onde de façon à ce que l'avertisseur sonore transmette les vibrations ultrasoniques générées par le transducteur au guide d'onde ; et
(d) un conduit (390)
accouplé au passage du guide d'onde, dans lequel le conduit est fonctionnel pour communiquer avec la ou les deux d'une source de vide ou d'une source de fluide pour transmettre l'un ou les deux d'une aspiration ou d'un fluide à travers le passage.

2. Système chirurgical selon la revendication 1, dans lequel le guide d'onde est constitué d'un seul composant monolithe formé en tant que continuum homogène de matière.

3. Système chirurgical selon la revendication 1, dans lequel l'effecteur d'extrémité est en communication fluidique avec le passage, de telle sorte que l'effecteur d'extrémité est fonctionnel pour transmettre l'un ou les deux d'une aspiration ou d'un fluide du passage à un site chirurgical.

4. Système chirurgical selon la revendication 1, dans lequel l'effecteur d'extrémité comporte une lame harmonique en communication acoustique avec le guide d'onde.

5. Système chirurgical selon la revendication 4, dans lequel la lame harmonique comporte une ou plusieurs ouvertures en communication fluidique avec le passage du guide d'onde.

6. Système chirurgical selon la revendication 5, dans lequel au moins une de l'une ou plusieurs ouvertures est positionnée sur un axe orienté transversalement par rapport à un axe longitudinal défini par la lame harmonique.

7. Système chirurgical selon la revendication 1, dans lequel l'extrémité proximale du guide d'onde comprend un cran, le conduit étant fixé au cran.

8. Système chirurgical selon la revendication 1, dans lequel le guide d'onde comprend un épaulement, l'avertisseur sonore étant mis en prise avec l'épaulement du guide d'onde.

9. Système chirurgical selon la revendication 1, dans lequel l'avertisseur sonore et le guide d'onde comprennent un filetage complémentaire, l'avertisseur sonore étant fixé au guide d'onde par le filetage complémentaire.

10. Système chirurgical selon la revendication 9, dans lequel l'écrou de compression et le guide d'onde comprennent un filetage complémentaire, l'écrou de compression étant fixé au guide d'onde par le filetage complémentaire.

11. Système chirurgical selon la revendication 1, comportant en outre une masse acoustique fixée au guide d'onde, la masse acoustique étant espacée du transducteur.

12. Système chirurgical selon la revendication 11, dans lequel la masse acoustique est située de manière proximale par rapport au transducteur.

13. Système chirurgical selon la revendication 11, dans lequel la masse acoustique et le guide d'onde comprennent un filetage complémentaire, la masse acoustique étant fixée au guide d'onde par le filetage complémentaire.

14. Système chirurgical selon la revendication 11, dans lequel la masse acoustique définit un passage en communication fluidique avec le passage du guide d'onde.

15. Système chirurgical selon la revendication 14, dans lequel le conduit est accouplé à la masse acoustique de telle sorte que le conduit est en communication fluidique avec le passage de la masse acoustique, la masse acoustique comprenant une caractéristique de retenue de conduit configurée pour fixer le conduit à la masse acoustique.

16. Système chirurgical selon la revendication 11, dans lequel la masse acoustique comprend une face distale, la face distale étant positionnée longitudinalement au niveau d'un noeud associé au guide d'onde.
